# EUROPEAN PATENT APPLICATION

(11) **EP 4 817 564 A1**
(43) Date of publication of application: **07.10.2026**
(21) Application number: 26170701.2
(22) Date of filing: 02.04.2026
(51) Int. Cl.: A61F 2/46

(54) **IMPLANT EXTRACTOR**

(30) Priority: 03.04.2025 US 202563782690 P
(71) Applicant: Shukla Medical, Saint Petersburg, Florida 33709 (US)
(72) Inventor: Paffrath, Colin, Apollo Beach, 33572 (US); Ahmed, Osama, Bulverde, 78163 (US)
(74) Representative: Cyrson, Matthew Dominic

(57) **Abstract**

An implant extractor including a housing, a shaft having a threaded proximal end and movable within the housing, a pair of jaws operatively connected to a distal end of the shaft for engaging an implant, and an adjuster having a threaded distal end operatively engaged with the threaded proximal end of the shaft and operable to move the shaft and the pair of jaws between a first position and a second position. In one aspect, operation of the adjuster, to move the shaft distally, along a longitudinal axis of the implant extractor, to move the pair of jaws from the first position to the second position, involves distal movement of the shaft pivotably rotating each jaw, about two axes, to move the pair of jaws from an open position to a closed position configured to clamp an implant to be extracted.

## Description

### CROSS-REFERENCE TO RELATED APPLICATION

This application claims the benefit of priority to U.S. Patent Application No. 63/782,690, filed April 3, 2025, entitled "IMPLANT EXTRACTOR," the entire disclosure of which is hereby incorporated by reference for all purposes.

### FIELD

The subject disclosure relates generally to surgical devices for removing implants and, more particularly, to a spine cage extractor.

### BACKGROUND

When performing a spinal surgical procedure to remove a spine cage implant from the spinal column, a surgeon must engage and extract the spine cage implant "free-hand" from between adjacent vertebrae. That is, the surgeon must first retract adjacent vertebrae to expose the spine cage and then insert a pliers type extraction device into the spinal column to engage the spine cage and, lastly, pull the spine cage from the spinal column. Accordingly, in free-hand extraction, a surgeon must physically squeeze an implant while also pulling the implant at the same time, which often leads to instability and loss of control during extraction. Therefore, disadvantages of free-hand spine cage implant extraction are potential risks of damage to the vertebrae on either side of the spine cage, to adjacent soft tissue, and to the spine cage itself.

An advantage exists for a spine cage extractor operable to extract spine cages with a high degree of stability and control, effectively reducing or eliminating free-hand movement. The implant extractor according to the subject disclosure effectively overcomes the disadvantages of free-hand spine cage extraction techniques.

### BRIEF SUMMARY OF THE DISCLOSURE

According to an exemplary embodiment of the subject disclosure, there is provided an implant extractor comprising a housing, a shaft having a threaded proximal end and movable within the housing, a pair of jaws operatively connected to a distal end of the shaft for engaging an implant, and an adjuster having a threaded distal end operatively engaged with the threaded proximal end of the shaft and operable to move the shaft and the pair of jaws between a first position and a second position.

In one aspect, operation of the adjuster, to move the shaft distally, along a longitudinal axis of the implant extractor, to move the pair of jaws from the first position to the second position, involves distal movement of the shaft pivotably rotating each jaw, about two axes, to move the pair of jaws from an open position to a closed position configured to clamp an implant to be extracted.

According to a further exemplary embodiment of the subject disclosure, there is provided an implant extractor comprising a shaft having a proximal end, a distal end, and an axially extending slot between the proximal and the distal ends; a housing surrounding the shaft, the housing having a proximal end, a distal end including bifurcated legs, and a radial opening aligned with the axially extending slot; a pin extending through the radial opening and the axially extending slot for coupling the shaft to the housing, whereby the axially extending slot permits relative axial movement between the shaft and the housing and the pin prevents rotational movement of the shaft relative to the housing; a pair of opposed jaws pivotably connected to the bifurcated legs; a first link pivotably connected to one of the pair of opposed jaws and to a plunger, where the plunger contacts the distal end of the shaft; a second link pivotably connected to the other of the pair of opposed jaws and to the plunger; and, a screw threadedly engaged, at a distal end thereof, to the proximal end of the shaft, and operable to move the shaft and the plunger distally relative to the housing to close the pair of jaws about an implant to be extracted.

In one aspect, the plunger can include an opening for receiving the distal end of the shaft; and/or the screw can comprise a first end including a socket, and a second threaded end threadedly received in an internally threaded proximal end of the shaft.

In another aspect, the implant extractor can further include a quick connect connected to the proximal end of the housing; and a power transfer member having a proximal end and a distal end, where the power transfer member can be releasably connectable to the quick connect. In this aspect, the distal end of the power transfer member can be configured to be received in the socket; and/or the proximal end of the power transfer member can be configured for connection to an extraction tool.

### BRIEF DESCRIPTION OF THE SEVERAL VIEWS OF THE DRAWINGS

The foregoing summary, as well as the following detailed description of the exemplary embodiments of the invention, will be better understood when read in conjunction with the appended drawings. For the purpose of illustrating the invention, there are shown in the drawings embodiments, which are presently preferred. It should be understood, however, that the invention is not limited to the precise arrangements and instrumentalities shown.

In the drawings:
FIG. 1 is a perspective view of an exemplary embodiment of an implant extractor of the subject disclosure;
FIG. 2 is an elevational cross-section view of the implant extractor of FIG. 1;
FIG. 3 is an elevational cross-section view of the implant extractor of FIG. 1 taken at 90 degrees with respect to the elevational cross-section view of FIG. 2;
FIG. 4 is an elevation view of a housing of the implant extractor of FIG. 1;
FIG. 5 is an elevation view of the housing of FIG. 4 taken at 90 degrees with respect to the elevational view of FIG. 4;
FIG. 6 is an elevational view of a shaft of the implant extractor of FIG. 1;
FIG. 7 is a perspective view of the shaft of FIG. 6;
FIG. 8 is an elevational cross-section view of the shaft of FIG. 6;
FIG. 9 is an elevational view of a plunger of the implant extractor of FIG. 1;
FIG. 10 is a perspective view of the plunger of FIG. 9;
FIG. 11 is an elevational view of a screw adjuster of the implant extractor of FIG. 1;
FIG. 12 is a perspective view of the screw adjuster of FIG. 11;
FIG. 13 is an elevational view of a power transfer member of the implant extractor of FIG. 1;
FIG. 14 is an elevation view of the power transfer member of FIG. 13 taken at 90 degrees with respect to the elevational view of FIG. 13;
FIG. 15 is a perspective cross-section view of a distal end of the implant extractor of FIG. 1;
FIG. 16 is an elevational cross-section view of the distal end of the implant extractor of FIG. 1; and
FIG. 17 is a perspective view of jaws of the implant extractor of FIG. 1.

### DETAILED DESCRIPTION

Reference will now be made in detail to the various embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the present disclosure can be practiced without one or more of the specific features or advantages of a particular exemplary embodiment. In other instances, additional features and advantages may be recognized in certain embodiments that may not be present in all exemplary embodiments of the subject disclosure.

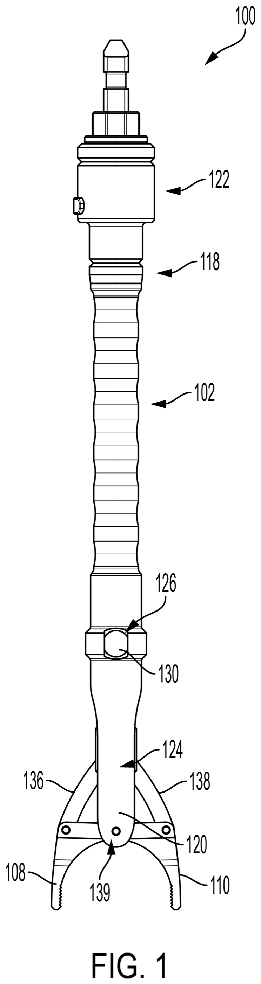

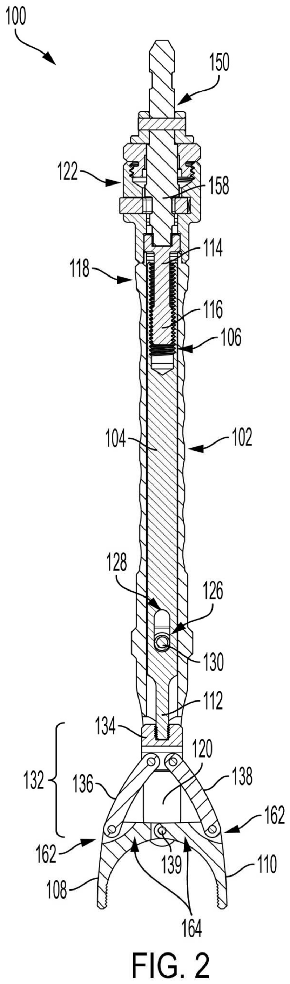

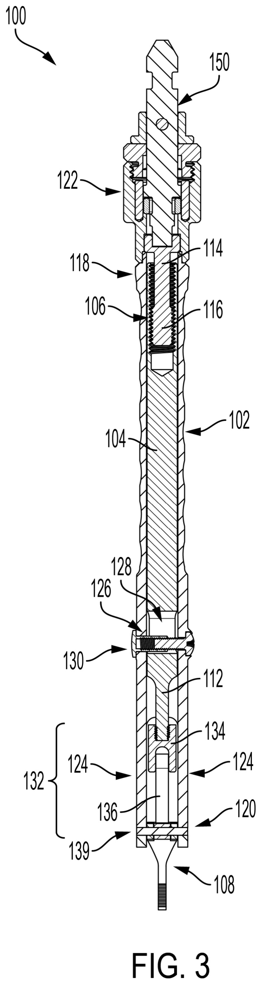

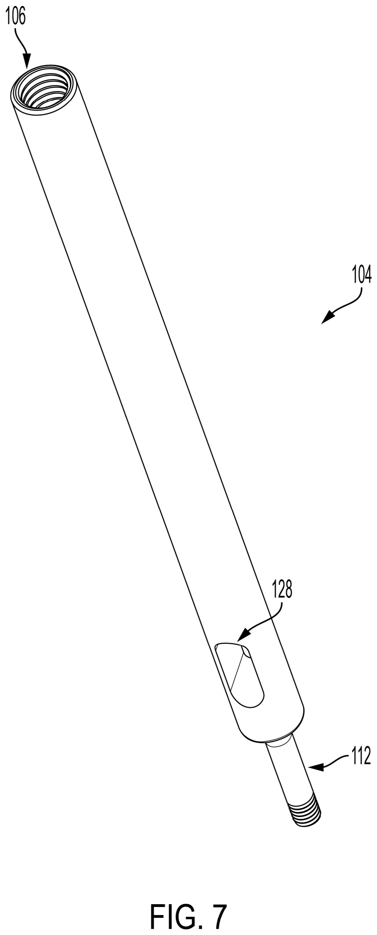

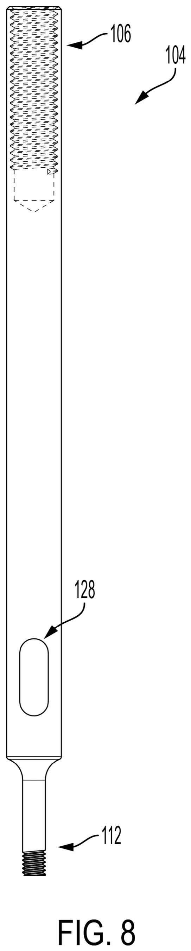

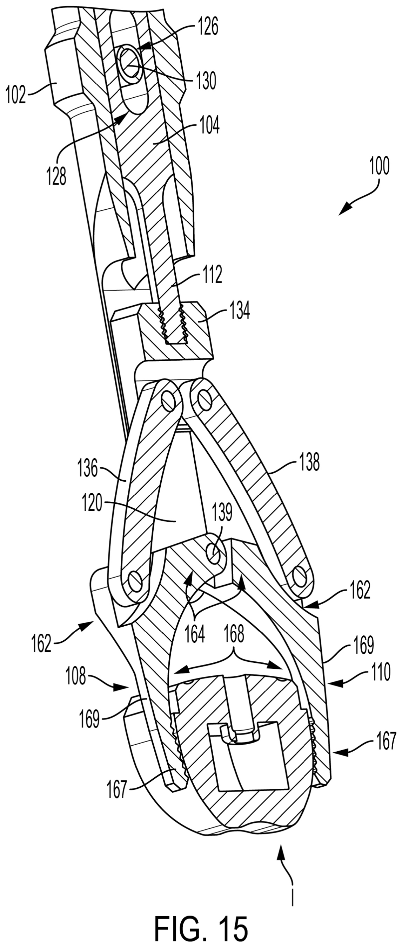

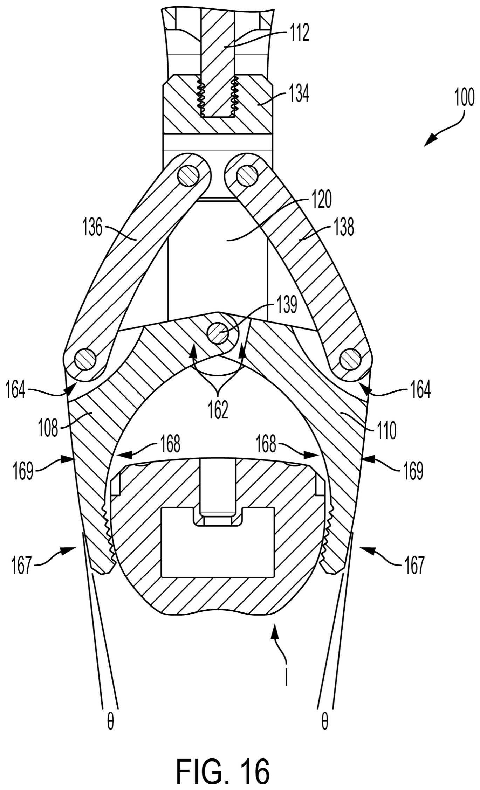

Referring to FIGS. 1-3, there is shown an implant extractor 100 constructed in accordance with an exemplary embodiment of the subject disclosure. The implant extractor 100 is suitable for use with extracting a spine cage implant but may be used to extract other types of bodily implants as well. The implant extractor comprises an elongated housing 102 surrounding a shaft 104 which is movable within the housing 102. As best shown in FIGS. 2, 3, 7 and 8, the shaft 104 has an internally threaded proximal end 106. The implant extractor 100 further comprises a pair of opposing jaws 108, 110 operatively connected to a distal end 112 of the shaft 104 and configured for selectively gripping an implant I (see FIGs 15 and 16). The implant extractor 100 additionally comprises an adjuster 114 having a threaded distal end 116 operatively engaged with the internally threaded proximal end 106 of the shaft 104, and operable to move the shaft 104 and the pair of jaws 108, 110 between a first position and a second position (e.g., between a position not engaging an implant and a position engaging an implant).

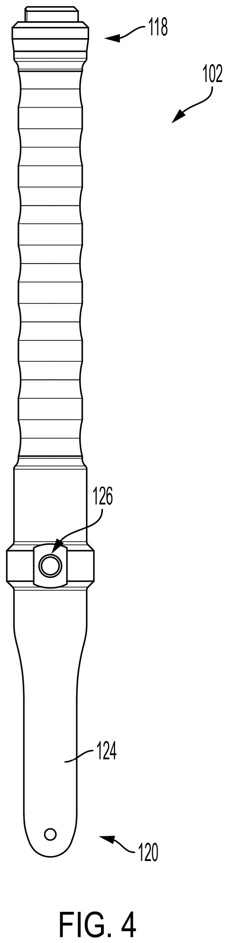

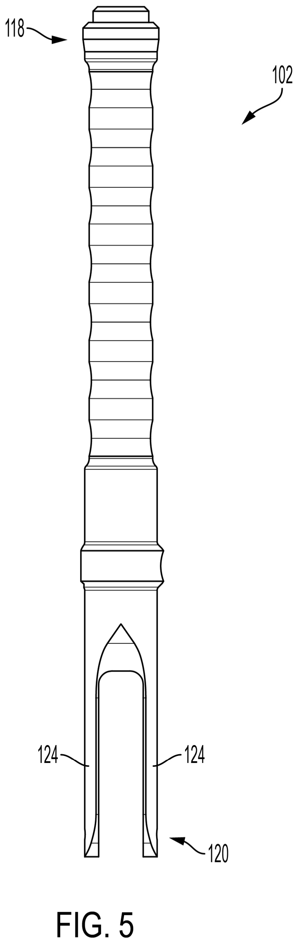

Referring to FIGS. 1-5, the housing 102 includes a proximal end 118 and a distal end 120. The proximal end 118 of the housing 102 is configured for attachment (e.g., by threading) to a quick connect 122 (FIGS. 1-3). The distal end 120 of the housing 102 includes bifurcated legs 124. The housing 102 further includes at least one radial opening 126 between the proximal 118 and the distal 120 ends.

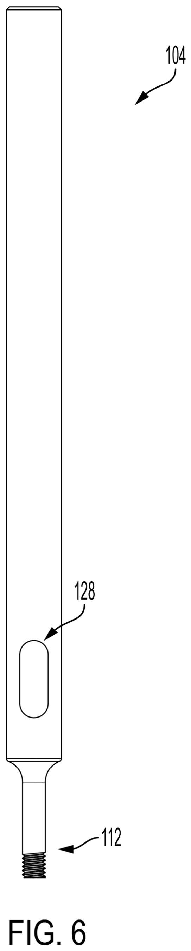

Referring to FIGS. 2, 3 and 6-8, the shaft 104 includes an axially extending slot 128 provided between the proximal 106 and the distal 112 ends of the shaft 104. The at least one radial opening 126 in the housing 102 is aligned with the axially extending slot 128.

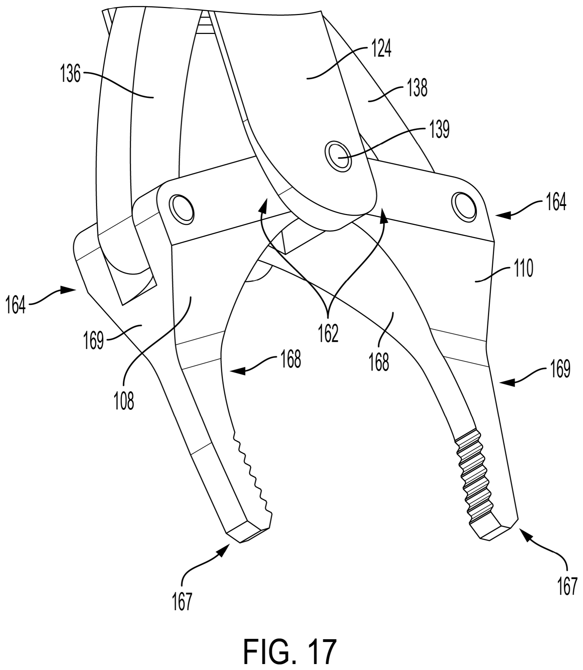

The implant extractor 100 further comprises a pin 130 (FIGS. 1-3) extending through the at least one radial opening 126 and through the axially extending slot 128 for operationally coupling the shaft 104 to the housing 102, where the axially extending slot 128 permits axial movement of the shaft 104 relative to the housing 102, and the pin 130 prevents rotational movement (resisting torque) of the shaft 104 relative to the housing 102. FIGS. 2, 3 and 15-17 further show a linkage 132 contacting the distal end 112 of the shaft 104, and operatively connected to the pair of jaws 108, 110.

According to an exemplary embodiment of the subject disclosure, the linkage 132 comprises a plunger 134 in contact with a distal end 112 of the shaft 104; a first link 136 pivotably connected to one of the pair of jaws (e.g., jaw 108) and to the plunger 134; and a second link 138 pivotably connected to the other of the pair of jaws (e.g., jaw 110) and to the plunger 134. Further, the pair of jaws 108, 110 are pivotably connected to the bifurcated legs 124 of the housing 102 at pivot 139 (FIGS. 1, 2 and 15-17).

In one embodiment, a distal end of the first link 136 is pivotably connected to a laterally positioned proximal end 162 of a first jaw 108, and the second link 138 is pivotably connected to a laterally positioned proximal end 162 of a second jaw 110. Further, medially positioned proximal ends 164 of each jaw 108, 110, respectively, are both pivotably connected to the bifurcated legs 124 of the housing 102 at the pivot 139 (FIGS. 1, 2 and 15-17).

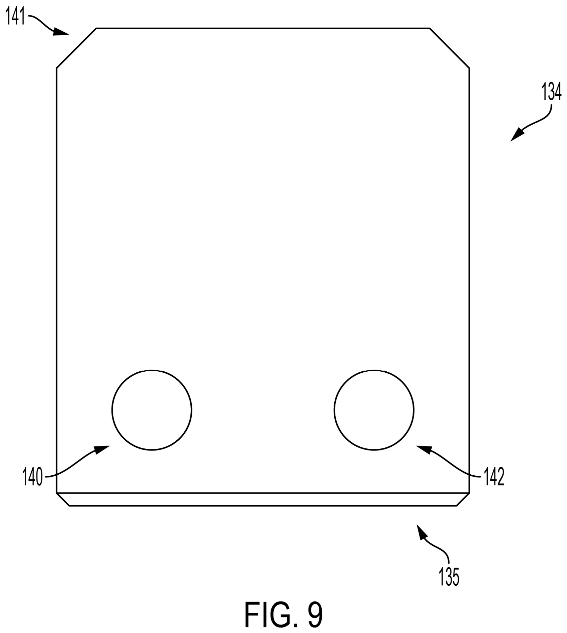

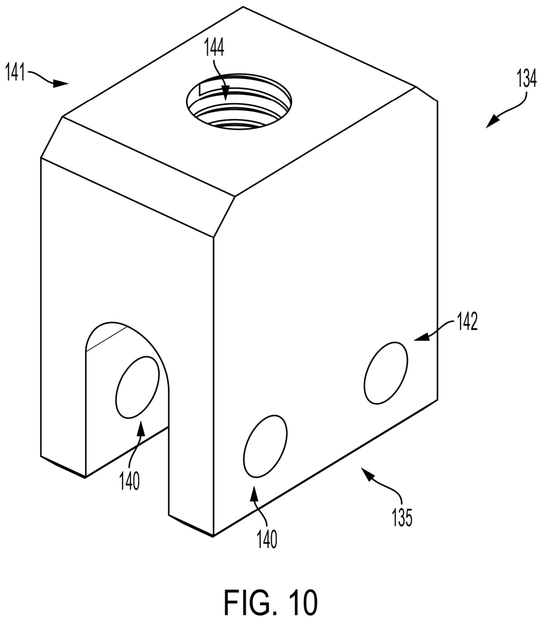

FIGS. 9 and 10 illustrate an exemplary construction of the plunger 134. Adjacent its distal end 135, the plunger 134 includes two pairs of aligned holes 140, 142 for pivotably connecting proximal ends of the first and the second links 136, 138, respectively, to the plunger 134. At its proximal end 141, the plunger 134 includes a blind hole or bore 144 sized to receive the distal end 112 of the shaft 104.

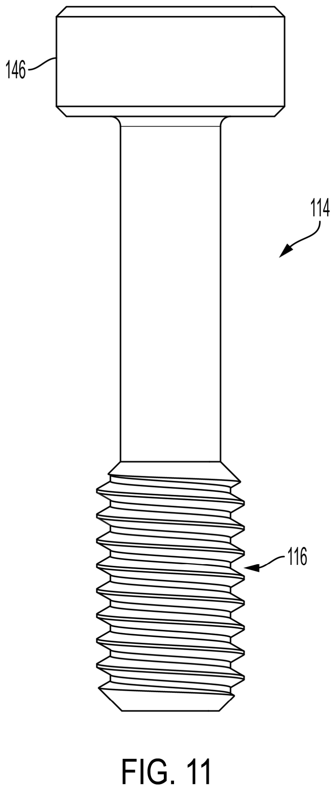

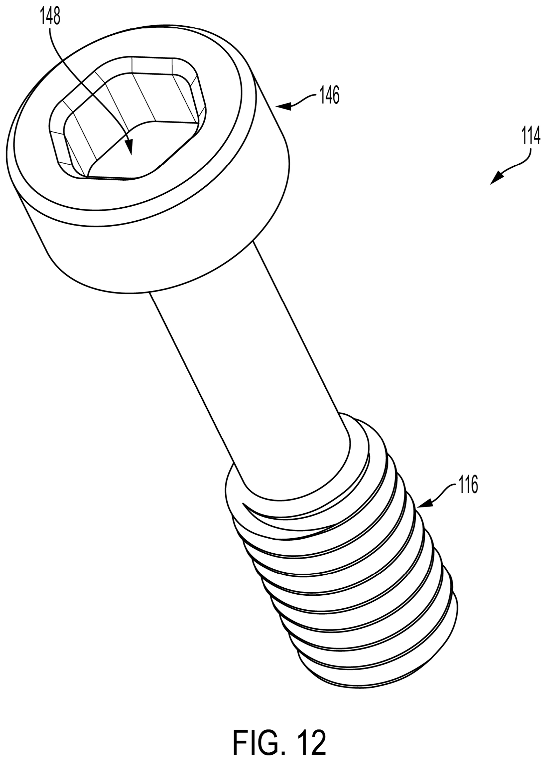

Referring to FIGS. 11 and 12, the adjuster 114 comprises a screw (or the like) and is shown to include the threaded distal end 116 operatively engaged with the threaded proximal end 106 of the shaft 104. The adjuster 114 screw further includes a proximal end 146 having a polygonal (e.g., hexagonal) socket 148 formed therein. The adjuster 114 screw is operable to move the shaft 104 and the plunger 134 distally relative to the housing 102 to close the pair of jaws 108, 110 about an implant.

In one aspect, the adjuster 114 screw turns (axially rotates) about, but remains translationally stationary relative to, a longitudinal axis of the implant extractor 100 (i.e., and a longitudinal axis of the housing 102, the shaft 104, and the adjuster 114). The turning adjuster 114 causes the (externally) threaded distal end 116 of the adjuster 114 to operationally engage the (internally) threaded proximal end 106 of the shaft 104 to translate (i.e., distally and proximally) the shaft 104 and the plunger 134 along the longitudinal axis of the implant extractor 100, relative to the housing 102, to operate (i.e., to close and to open) the pair of jaws 108, 110 around an implant (see FIGS. 15-17). The adjuster screw 114 is turned by a power transfer member described below.

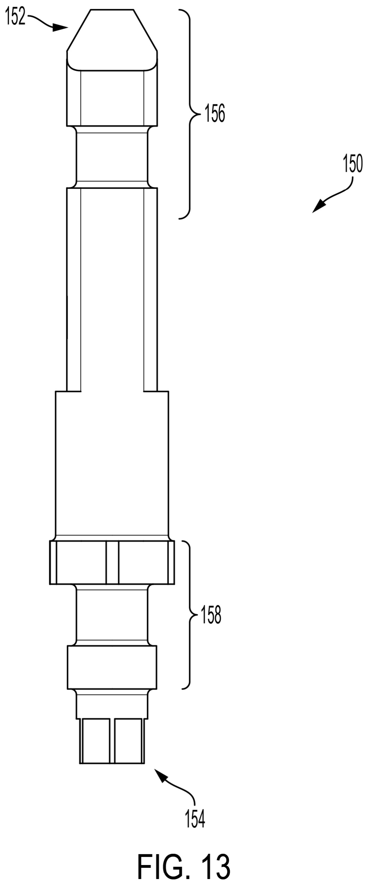

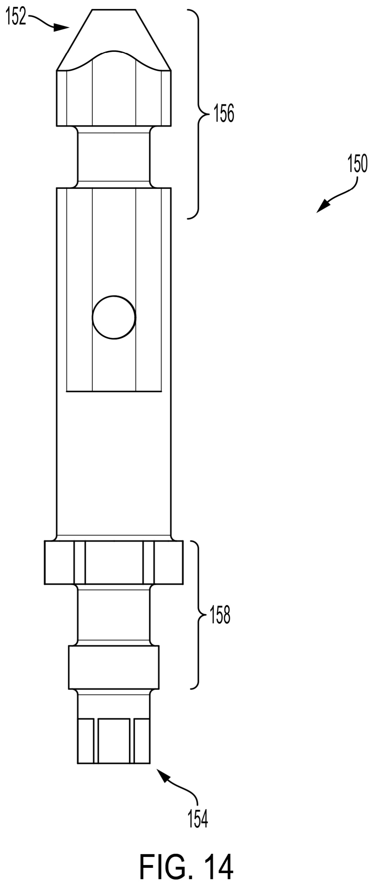

FIGS. 13 and 14 illustrate an exemplary construction of a power transfer member 150 for turning the adjuster screw 114. The power transfer member 150 includes a proximal end 152 and a distal end 154. The power transfer member 150 includes a first quick connection (e.g., male quick connection) 156 adjacent the proximal end 152 thereof configured for releasable connection to an unillustrated turnable extraction tool (e.g., a T-handle or the like). The power transfer member 150 further includes a second quick connection (e.g., male quick connection) 158 adjacent the distal end 154 thereof for releasably connecting the power transfer member 150 to the quick connect 122 for rotation therewithin. The distal end 154 is configured to be received in the of the socket 148 of the adjuster 114 screw. According to one aspect, the distal end 154 is configured as a polygonal (e.g., hexagonal) head suited for releasable mating engagement with the adjuster 114 screw socket 148.

Prior to using the implant extractor 100, the second quick connection 158 of the power transfer member 150 is connected to the quick connect 122 such that the distal end 154 of the power transfer member is received in the adjuster 114 screw socket 148. A turnable extraction tool is then connected to the first quick connection 156 of the power transfer member 150. With the opposed jaws 108, 110 in a first (or open) position, wider than an implant I to be extracted, a surgeon places distal ends 167 (and/or medial sides 168) of the opposed jaws 108, 110 adjacent opposite sides of the implant I and commences turning the extraction tool. As the extraction tool is turned, so too is the adjuster screw 114, which causes the shaft 104 to axially move relative to the housing 102 by virtue of the axial slot 128 moving axially about the pin 130 (recall that any rotational torque of the shaft 104 relative to the housing 102 is prevented by the pin 130, which non-rotatably maintains the shaft 104 and the housing 102 together, providing counter-torque on the shaft 104 to prevent any unnecessary forces on the implant I to be removed, as the jaws 108, 110 are tightened).

As the shaft 104 moves axially (translates) in a distal direction, the linkage 132 causes the jaws 108, 110 to close about the implant I until the jaws 108, 110 firmly grip the implant (see FIGS. 15 and 16). More particularly, distal movement of the shaft 104 relative to the housing 102 urges the plunger 134 and the first and the second links 136, 138 distally, where a spacing between the distal ends 167 of the jaws 108, 110 is reduced until the jaws 108, 110 firmly grip the implant. That is, because of the pivotal connection of a medially located, proximal end 164, 165 of each jaw 108, 110 to distal ends of the bifurcated legs 124 of the housing 102 at pivot 139 (see FIGS. 1 and 2), distally directed translation of the plunger 134 and, thereby, of laterally located proximal ends of each of the first and the second links 136, 138, causes jaw 108, 110 rotation about pivot 139, and a closing of the space between the distal ends 167 of the jaws 108, 110 to grip the implant. Once the implant I is gripped, the surgeon may simply pull on the turning tool (or the implant extractor 100 generally) to extract the implant.

The jaws 108, 110 can each have medial sides 168, and/or a distal end 167, that are textured to better grip an implant I (e.g., textured to include ribs, teeth, knurling, protrusions, grooves, ridges, dimples, spikes, or a combination thereof). Further, the jaws 108, 110 can be angled-in slightly to draw the implant I closer and to prevent slippage. That is, a lateral side 169 of each jaw 108, 110 can taper inwardly, toward a longitudinal axis of the of the implant extractor 100, while moving distally from the laterally positioned proximal end 162 to the distal end/tip 167 of each jaw 108, 110. This inward taper may only occur, or more particularly may occur, over a distal end 167 of each jaw 108, 110. Over this distal end 167 of each jaw, the medial side 168 of each jaw 108, 110 may also be tapered inwardly, or be similarly tapered inwardly (i.e., similar to the taper of the respective lateral side 169). Otherwise, the medial sides 168 of each jaw 108, 100 generally have a concave contour from distal end 167 to the medially positioned proximal end 164 thereof. The jaws 108, 100 function to allow multiple sizes of implants (e.g., spine cages) to be extracted based on a degree of force applied by an extraction tool (e.g., a T-handle or the like) releasably connected to the power transfer member 150.

In conventional free-hand extraction methods a surgeon uses a hand-held pliers-type tool to physically squeeze an implant while pulling at the same time, which often leads to instability during extraction. In contrast, once the jaws 108, 110 of the implant extractor 100 of the subject disclosure are clamped to an implant, a surgeon does not have to squeeze the implant while withdrawing the implant, thereby resulting in considerably improved stability and less harm to a patient during an extraction process.

In addition, once the jaws 108, 110 of the implant extractor 100 are clamped to an implant I, a strike plate/slide hammer can be attached to the implant extractor 100 for inline removal of the implant. Any axial motion of the implant extractor 100 resulting from an attached power transfer member, is transferred to the housing 102, while the shaft 104 and the jaws 108, 110 remain fixed on the implant. A stable clamping effect is achieved as the housing 102 and the shaft 104 move oppositely and tighten on an implant.

It will be appreciated by those skilled in the art that changes could be made to the various aspects described above without departing from the broad inventive concept thereof. It is to be understood, therefore, that the subject application is not limited to the particular aspects disclosed, but it is intended to cover modifications within the spirit and scope of the subject application as defined by the appended claims.

## Claims

1. An implant extractor (100) **characterized in that**, and comprising:
a housing (102);
a shaft (104) movable within the housing (102), the shaft (104) having a proximal end (106);
a pair of jaws (108, 110) operatively connected to a distal end (112) of the shaft (104); and
an adjuster (114) having a distal end (116) operatively engaged with the proximal end (106) of the shaft (104), the adjuster (114) being operable to move the shaft (104) and the pair of jaws (108, 110) between a first position and a second position.

2. The implant extractor (100) of claim 1, further comprising an axially extending slot (128) provided between the proximal and distal ends of the shaft (104).

3. The implant extractor (100) of claim 2, further comprising a radial opening (126) in the housing (102) aligned with the axially extending slot (128).

4. The implant extractor (100) of claim 3, further comprising a pin (130) extending through the radial opening (126) and the axially extending slot (128) for coupling the shaft (104) to the housing (102), whereby the axially extending slot (128) permits axial movement of the shaft (104) relative to the housing (102) and the pin (130) therethrough prevents rotational movement of the shaft (104) relative to the housing (102).

5. The implant extractor (100) of any preceding claim, further comprising a linkage (132) contacting the distal end (112) of the shaft (104) and operatively connected to the pair of jaws (108, 110).

6. The implant extractor (100) of claim 5, wherein the linkage (132) comprises:
a plunger (134) in contact with the distal end (112) of the shaft (104);
a first link (136) pivotably connected to one of the pair of jaws (108, 110) and to the plunger (134); and
a second link (138) pivotably connected to the other of the pair of jaws (108, 110) and to the plunger (134).

7. The implant extractor (100) of claim 6, wherein the plunger (134) includes an opening (144) for receiving the distal end (112) of the shaft (104).

8. The implant extractor (100) of any preceding claim, wherein a distal end of the housing (102) includes bifurcated legs (124) and the pair of jaws (108, 110) is pivotably connected to the bifurcated legs (124).

9. The implant extractor (100) of any preceding claim, wherein the adjuster (114) comprises a proximal end including a socket (148), and a threaded distal end (116) threadedly engaging a threaded proximal end (106) of the shaft (104).

10. The implant extractor (100) of any preceding claim, further comprising:
a quick connect (122) connected to a proximal end (118) of the housing (102); and
a power transfer member (150) having a proximal end (152) and a distal end (154), wherein the power transfer member (150) is releasably connectable to the quick connect (122).

11. The implant extractor (100) of claim 10, wherein the distal end (154) of the power transfer member (150) is configured to be received in a socket (148) in a proximal end of the adjuster (114) and the proximal end (152) of the power transfer member (150) is configured for connection to an extraction tool.

12. The implant extractor (100) of any preceding claim, wherein operation of the adjuster (114), to move the shaft (104) distally, along a longitudinal axis of the implant extractor (100), to move the pair of jaws (108, 110) from the first position to the second position, involves distal movement of the shaft (104) to pivotably rotate each jaw (108, 110), about two axes, to move the pair of jaws (108, 110) from an open position to a closed position, the closed position configured to clamp an implant to be extracted.

13. The implant extractor (100) of claim 1, wherein:
the shaft (104) further includes a distal end (112), and an axially extending slot (128) between the proximal and the distal ends;
the housing (102) surrounds the shaft (104), the housing (102) having a proximal end, a distal end including bifurcated legs, and a radial opening (126) aligned with the axially extending slot (128);
the implant extractor further includes a pin (130) extending through the radial opening (126) and the axially extending slot (128) for coupling the shaft (104) to the housing (102), whereby the axially extending slot (128) permits relative axial movement between the shaft (104) and the housing (102) and the pin (130) prevents rotational movement of the shaft (104) relative to the housing (102);
the pair of jaws (108, 110) are opposed, and are pivotably connected to the bifurcated legs;
the implant extractor further includes a first link (136) pivotably connected to one of the pair of opposed jaws and to a plunger (134), wherein the plunger (134) includes an opening for receiving the distal end (112) of the shaft (104);
the implant extractor further includes a second link (138) pivotably connected to the other of the pair of opposed jaws and to the plunger (134); and
the adjuster (114) is threadedly engaged, at a distal end (116) thereof, to the proximal end of the shaft (104), the adjuster (114) being operable to move the shaft (104) and the plunger (134) distally relative to the housing (102) to close the pair of opposed jaws about an implant to be extracted.

14. The implant extractor (100) of claim 13, wherein the adjuster (114) comprises a first end including a socket (148), and a second threaded end threadedly received in an internally threaded proximal end of the shaft (104).

15. The implant extractor (100) of claim 14, further comprising:
a quick connect (122) connected to the proximal end of the housing (102); and
a power transfer member (150) having a proximal end (152) and a distal end (154), wherein:
the power transfer member (150) is releasably connectable to the quick connect (122);
the distal end (154) of the power transfer member (150) is configured to be received in the socket (148); and
the proximal end (152) of the power transfer member (150) is configured for connection to an extraction tool.
